# EUROPÄISCHE PATENTSCHRIFT

(11) **EP 3 513 753 B1**
(45) Veröffentlichungstag und Bekanntmachung des Hinweises auf die Patenterteilung: **15.03.2023**
(21) Anmeldenummer: 19152369.5
(22) Anmeldetag: 17.01.2019
(51) Int. Cl.: A61B 17/34, A61B 5/00

(54) **INJEKTOR ZUM TRANSKUTANEN EINFÜHREN EINES SENSORS IN EINEN PATIENTEN**
INJECTOR FOR TRANSCUTANEOUS INSERTION OF A SENSOR IN A PATIENT
INJECTEUR DESTINÉ À L'INTRODUCTION TRANSCUTANE D'UN CAPTEUR DANS UN PATIENT

(30) Priorität: 22.01.2018 DE 102018101283
(43) Veröffentlichungstag der Anmeldung: 24.07.2019
(73) Patentinhaber: EyeSense GmbH, 63762 Großostheim (DE)
(72) Erfinder: Müller, Achim, 63762 Großostheim (DE); Meissner-Braun, Tom, 88662 Überlingen (DE); Pischan, Matthias, 64287 Darmstadt (DE)
(74) Vertreter: LBP Lemcke, Brommer & Partner Patentanwälte mbB

(56) Entgegenhaltungen:
- EP-A1- 2 452 638
- DE-B3-102018 101 313
- US-A1- 2004 133 164
- US-A1- 2008 249 466
- US-A1- 2011 040 245
- US-A1- 2016 243 302
- US-A1- 2017 028 128
- US-A1- 2017 303 831

## Beschreibung

Die Erfindung betrifft einen Injektor zum transkutanen Einführen eines Sensors in einen Patienten gemäß Oberbegriff des Anspruchs 1. Für vielfältige medizinischen Anwendungen ist das Einführen eines Sensors in einen Patienten notwendig, insbesondere, um Messwerte des Patienten zu erhalten, wie beispielsweise Glukosewerte oder Laktosewerte.

Aus US 8,029,442 B2 ist ein Injektor zum transkutanen Einführen eines Sensors in einen Patienten bekannt, bei welchem eine geschlitzte Kanüle und ein sich darin befindender Sensor mittels eines Schiebeelementes transkutan in einen Patienten eingeführt werden. Mittels einer Ejektionsfeder wird die Kanüle nach Beenden des Injektionsvorgangs wieder aus dem Patienten herausgezogen.

Aus US 2016/0243302 A1 ist ein Injektor zum subkutanen Einführen medizinischer Geräte über eine Injektionsnadel bekannt. Die Injektionsnadel ist an einem Teil des Injektors angeordnet, welcher über eine Feder automatisch aus dem Gewebe des Patienten herausgezogen werden kann.

Aus US 2017/0303831 A1 ist ein Injektor zum Einführen von Sensoren in Patienten bekannt. Zur Durchführung eines Injektionsvorganges wird der Injektor mit einer Basisplatte verbunden, die am Patienten angeordnet ist. Nach Abschluss des Injektionsvorganges wird die Nadel aus dem Patienten herausgezogen.

Aus EP 2 452 638 A1 ist eine Vorrichtung zum Injizieren eines Feststoffes in einen menschlichen oder tierischen Körper bekannt. Die Vorrichtung umfasst einen Spritzenkörper, der in einem Gehäuse der Vorrichtung gelagert ist und über eine Federkraft aus dem Körper herausgezogen werden kann.

Aus US 2011/040245 A1 ist ein Injektor mit einem Ejektionselement bekannt, der während eines Injektionsvorganges Energie aufnimmt und unmittelbar nach Abschluss des Injektionsvorganges eine Ejektionsbewegung einer Injektionsnadel aus dem Gewebe eines Patienten bewirkt.

Aus US 2008/249466 A1 ist ein Injektor mit einem Ejektionselement bekannt, mit dem eine Injektion eines Sensors in einen Patienten durchgeführt werden kann. Der bekannte Injektor kann flexibel am Patienten positioniert werden.

Aus US 2017/028128 A1 ist eine Einführungsvorrichtung für einen Katheter zum Anschließen an eine Insulinpumpe bekannt, wobei die Vorrichtung ein Gehäuse mit einem Basiselement, einen Katheter, eine Einführungsnadel und ein im Gehäuse montiertes Injektionselement umfasst. Mittels des Injektionselements wird der Katheter und die Nadel, welche miteinander gekoppelt sind in bzw. unter die Haut eines Patienten injiziert.

Der vorliegenden Erfindung liegt die Aufgabe zugrunde, einen Injektor zum transkutanen Einführen eines Sensors in einen Patienten zu schaffen, der die Sicherheit beim Injektionsvorgang erhöht und den Anwendungskomfort für den Benutzer erhöht.

Gelöst ist diese Aufgabe durch einen Injektor gemäß Anspruch 1. Vorteilhafte Ausgestaltungen finden sich in den abhängigen Unteransprüchen.

Der erfindungsgemäße Injektor zum transkutanen Einführen eines Sensors in einen Patienten weist eine Kanüle, ein Basiselement, ein verschiebbar an dem Basiselement angeordnetes Schiebeelement zum transkutanen Einführen der Kanüle in den Patienten in einer Injektionsrichtung auf und ein Ejektionselement zum selbsttätigen Herausziehen der Kanüle aus dem Patienten entgegen der Injektionsrichtung mittels des Ejektionselementes in einem Ejektionsvorgang, wobei der Injektor ein Arretierungselement für das Ejektionselement aufweist, sodass in einem Auslieferungszustand das Ejektionselement in einem energiegeladenen Zustand arretierbar ist und das Schiebeelement und Arretierungselement mittelbar oder unmittelbar zusammenwirkend ausgebildet sind, um in einem Injektionszustand bei in dem Patienten transkutan eingeführter Kanüle die Arretierung des Ejektionselementes zu lösen, um den Ejektionsvorgang automatisch zu starten.

Wesentlich ist, dass das Arretierungselement ist in einer Festlegestellung an dem Basiselement festlegbar ausgebildet und in einer Ejektionsstellung entgegen der Injektionsrichtung verschiebbar ausgebildet ist und dass das Arretierungselement drehbar an dem Basiselement angeordnet ist und mittels Drehung des Arretierungselementes aus der Festlegestellung in die Ejektionsstellung überführbar ausgebildet ist.

Der erfindungsgemäße Injektor ermöglicht somit nach Beenden des Injektionsvorgangs ein automatisches Starten des Ejektionsvorgangs. Darüber hinaus wird die benötigte Energie für die Ejektion, das heißt das Herausziehen der Kanüle aus dem Patienten, durch das Ejektionselement zur Verfügung gestellt. Hierdurch wird in einem Auslieferungszustand das Ejektionselement in einem energiegeladenen Zustand arretiert. Schiebeelement und Arretierungselement sind zusammenwirkend ausgebildet, sodass im Injektionszustand, bei in den Patienten transkutan eingeführter Kanüle, das Arretierungselement lösbar ist, sodass automatisch mittels der in dem Ejektionselement gespeicherten Energie die Ejektion, das heißt das Herausziehen der Nadel, erfolgt.

Der Injektor weist den Vorteil auf, dass durch den Benutzer der Ejektionsvorgang nicht manuell durchgeführt werden muss. Hierdurch ist insbesondere kein Umgreifen oder eine manuelle Richtungsänderung nach Abschluss des Injektionsvorgangs notwendig, sodass Stöße oder ein Verschieben des Injektors, die zu Schmerzen des Patienten, einer fehlerhaften Platzierung des Sensors oder einer Dejustierung des Injektors führen können, vermieden werden. Weiterhin wird die notwendige Energie für den Ejektionsvorgang durch die im Auslieferungszustand gespeicherte Energie des Ejektionselementes vorgegeben. Es ist somit nicht notwendig, dass beispielsweise der Benutzer während eines manuellen Injektionsvorgangs gleichzeitig beispielsweise durch Komprimieren einer Feder die Energie für einen Ejektionsvorgang zur Verfügung stellt. Der durch den Benutzer während des Injektionsvorgangs auszuübende Druck wird daher bei der vorliegenden Erfindung nicht durch die notwendige Energie bzw. Kraft für den Ejektionsvorgang bestimmt, sondern kann auf ein sicheres Durchführen des Injektionsvorgangs abgestimmt werden. Insbesondere wird bei der vorliegenden Erfindung vermieden, dass aufgrund eines hohen Kraftaufwandes ein Injektionsvorgang - beispielsweise zum Spannen einer Ejektionsfeder - ein Verkippen oder Verschieben des Injektors erfolgt, welches zu Unannehmlichkeiten für den Patienten bis hin zu Verletzungen oder fehlerhaft eingeführten Sensoren führen kann.

Durch die Festlegbarkeit des Arretierungselements in einer Festlegestellung an dem Basiselement und eine Verschiebbarkeit des Kanülenoberteils entgegen der Injektionsrichtung in einer Ejektionsstellung ergibt sich eine konstruktiv einfache Ausgestaltung. Im Auslieferungszustand wird das Ejektionselement in den energiegeladenen Zustand gebracht und anschließend das Arretierungselement in der Festlegestellung angeordnet, sodass die Energie des Ejektionselementes gespeichert bleibt. Weiterhin ist in dieser bevorzugten Ausführungsform der Injektor derart ausgebildet, dass im Injektionszustand mittels des Schiebeelementes das Arretierungselement aus der Festlegestellung in die Ejektionsstellung gebracht wird. In dieser Ejektionsstellung ist das Arretierungselement nun verschiebbar entgegen der Injektionsrichtung, sodass während des Ejektionsvorgangs Arretierungselement, Kanüle und gegebenenfalls Kanülenoberteil entgegen der Injektionsrichtung mittels der in dem Ejektionselement gespeichert Energie verschoben werden, sodass die Kanüle aus dem Patienten herausgezogen wird.

Vorteilhafterweise ist die Kanüle in einem distalen Endbereich an einem Kanülenoberteil des Injektors angeordnet. Das Kanülenoberteil ist in Injektionsrichtung verschiebbar an dem Basiselement angeordnet und das Ejektionselement ist mit dem Kanülenoberteil zusammenwirkend ausgebildet, sodass mittels des Ejektionselementes das Kanülenoberteil entlang der Injektionsachse entgegen der Injektionsrichtung verschiebbar ist.

Hierdurch ergibt sich eine konstruktiv einfache Ausgestaltung, da sowohl bei Injektion als auch bei Ejektion die Krafteinleitung in die Kanüle über das Kanülenoberteil erfolgen kann und insbesondere eine definierte Angriffsfläche - mittelbar oder bevorzugt unmittelbar - für das Ejektionselement gegeben ist, um die Kraft entgegen der Injektionsrichtung über das Kanülenoberteil zu der Kanüle zu leiten, um die Kanüle aus dem Patienten herauszuziehen.

Hierbei ist es vorteilhaft, dass das Arretierungselement um eine Injektionsachse drehbar an dem Basiselement angeordnet ist.

Der Injektor ist derart ausgebildet, dass die Kanüle mittels des Schiebeelementes in eine Injektionsrichtung in den Patienten transkutan eingeführt wird. Dies erfolgt bevorzugt entlang einer geradlinigen Injektionsachse. Diese Injektionsachse kann schräg zu der Oberfläche des Gewebes des Patienten stehen. Besonders vorteilhaft ist es, dass die Injektionsachse senkrecht zu der Oberfläche des Gewebes des Patienten steht, in welches der Sensor eingeführt werden soll.

In der vorbeschriebenen vorteilhaften Ausführungsform ist das Arretierungselement wie zuvor beschrieben um die Injektionsachse drehbar ausgebildet. Hierdurch ergibt sich eine konstruktiv einfache Ausgestaltung, da ein Verschieben des Arretierungselementes während des Ejektionsvorgangs entlang der Injektionsachse entgegen der Injektionsrichtung erfolgt und ein Überführen des Arretierungselementes aus der Festlegestellung in die Ejektionsstellung mittels einer Drehung um die Injektionsachse erfolgt. Hierdurch kann in konstruktiv einfacher Ausgestaltung das Lösen des Arretierungselementes erfolgen, insbesondere durch Vorsehen von Schrägen an Schiebeelement und/oder Arretierungselement, wie nachfolgend näher ausgeführt.

Vorteilhafterweise ist das Arretierungselement zwischen Ejektionselement und Kanülenoberteil oder zumindest Angriffsflächen des Kanülenoberteils für das Ejektionselement angeordnet, sodass mittels des Ejektionselementes eine Kraft in Ejektionsrichtung über das Arretierungselement auf das Kanülenoberteil übertragbar ist.

In dieser vorteilhaften Ausgestaltung ergibt sich somit weiterhin eine konstruktive Vereinfachung, dass das Ejektionselement einerseits durch das Arretierungselement in Festlegezustand arretiert ist, andererseits im Ejektionszustand das Ejektionselement mittels Krafteinwirkung auf das Arretierungselement in konstruktiv einfacher Weise eine Krafteinwirkung für den Ejektionsvorgang ausübt, wobei die Kraft entgegen der Ejektionsrichtung von dem Ejektionselement über das Arretierelement auf das Kanülenoberteil und somit auf die Kanüle zum Herausziehen der Kanüle aus dem Patienten übertragen wird.

Das Ejektionselement kann in unterschiedlicher Weise ausgebildet sein, um Energie für den Ejektionsvorgang zu speichern. Es liegt im Rahmen der Erfindung, das Ejektionselement als Druckspeicher auszubilden, in welchem ein Überdruck oder ein Unterdruck gegenüber dem Atmosphärendruck gespeichert wird. Das Arretierungselement ist hierbei als Verschlusselement für das als Druckspeicher ausgebildete Ejektionselement ausgeführt. In der Festlegestellung verschließt das Arretierungselement den Druckspeicher. In der Ejektionsstellung ist eine Ausgabeöffnung des Druckspeichers, welche zuvor mittels des Arretierungselementes verschlossen war, geöffnet. Über Druckkanäle wird ein Druckausgleich unter Verschieben der Kanüle, insbesondere Verschieben des Kanülenoberteils, bevorzugt des Arretierungselementes erzielt.

Bevorzugt ist das Ejektionselement als Feder (Ejektionsfeder) ausgebildet. Hierdurch wird in konstruktiv einfacher Weise eine Energiespeicherung ermöglicht, indem im Auslieferungszustand die Feder komprimiert oder gestreckt festgelegt ist. Entsprechend ist das Arretierungsmittel ausgebildet, in Festlegestellung die Feder im komprimierten oder gestreckten Zustand festzulegen und in Ejektionsstellung ist diese Festlegung aufgehoben, sodass eine Expansion oder Kompression der Feder erfolgt, welche zumindest mittelbar zu einem Herausziehen der Kanüle aus dem Patienten führt.

Eine besonders konstruktiv einfache vorteilhafte Ausgestaltung ergibt sich, indem die Feder im komprimierten Zustand festgelegt ist, sodass in diesem Auslieferungszustand eine Kraft zwischen Basiselement und an dem Basiselement in Festlegestellung angeordnetem Arretierungselement anliegt. In diesem Fall steht somit die in der komprimierten Feder gespeicherte Energie für die Ejektion zur Verfügung. Nach Beenden des Injektionsvorgangs erfolgt ein Überführen des Arretierungsmittels aus der Festlegestellung in die Ejektionsstellung, in welcher keine Festlegung der Feder mehr vorliegt, sodass eine Expansion der Feder erfolgt, welche zum Herausziehen der Kanüle aus dem Patienten verwendet wird.

Hier ist es insbesondere vorteilhaft, wie zuvor beschrieben, das Arretierungselement entlang dem Basiselement verschiebbar auszubilden und insbesondere bevorzugt das Arretierungselement um die Injektionsachse drehbar zum Überführen von der Festlegestellung in die Ejektionsstellung auszubilden.

Eine weiterhin konstruktiv einfache Ausgestaltung ergibt sich, indem die Feder einen zylindrischen Aufbau aufweist, insbesondere mit einem ovalen, bevorzugt kreisförmigen Querschnitt, und die Mittelachse der Feder parallel zur Injektionsachse angeordnet ist, insbesondere, indem die Kanüle im Bereich der Mittelachse der Feder angeordnet ist.

Wie zuvor beschrieben, weist der Injektor bevorzugt ein Kanülenoberteil auf, an welchem die Kanüle mit einem distalen Ende angeordnet ist. Insbesondere ist es vorteilhaft, dass das Schiebeelement und Kanülenoberteil derart zusammenwirkend ausgebildet sind, dass bei dem Injektionsvorgang das Kanülenoberteil mittels des Schiebeelements in Injektionsrichtung verschiebbar ist. Die Krafteinwirkung auf die Kanüle erfolgt in dieser bevorzugten Ausführungsform somit mittelbar, indem der Benutzer das Schiebeelement in Richtung des Patienten verschiebt und hierdurch eine Krafteinwirkung über das Kanülenoberteil auf die Kanüle erfolgt, um die Kanüle transkutan in den Patienten einzubringen.

Die Kanüle ist bevorzugt fest, insbesondere bevorzugt unlösbar mit dem Oberteil verbunden. Vorteilhafterweise umschließt das Kanülenoberteil die Kanüle an einem distalen Ende der Kanüle.

Das Kanülenoberteil weist bevorzugt ein Zentralelement und mindestens einen Führungsfortsatz auf. Das Basiselement weist bevorzugt mindestens eine Führungswand mit einem Führungsschlitz für den Führungsfortsatz des Kanülenoberteils auf, zur Führung des Kanülenoberteils in Injektionsrichtung. Die Kanüle ist bei dieser vorteilhaften Ausgestaltung an dem Zentralelement angeordnet. Vorteilhafterweise greift das Schiebeelement an den Führungsfortsatz an, sodass die Kraftübertragung bei Betätigen des Schiebeelementes über den Führungsfortsatz auf das Zentralelement auf die Kanüle erfolgt. Insbesondere ist es vorteilhaft, dass der Führungsfortsatz die Führungswand des Basiselementes durchdringt und das Schiebeelement an der dem Zentralelement des Kanülenoberteils abgewandten Seite der Führungswand an den Führungsfortsatz angreifend ausgebildet ist. Hierdurch ist eine effektive Führung des Kanülenoberteils in Injektionsrichtung durch die Führungswand und den Führungsschlitz in der Führungswand gegeben.

In einer vorteilhaften Weiterbildung wird ein Verkippen des Kanülenoberteils bei Verschieben in Injektionsrichtung vermieden, indem an zwei gegenüberliegenden Seiten jeweils ein Führungsfortsatz an dem Kanülenoberteil ausgebildet ist und das Basiselement korrespondierend zumindest zwei Führungsschlitze in Injektionsrichtung für die beiden Führungsfortsätze aufweist und das Schiebeelement an beiden Führungsfortsätzen angreifend ausgebildet ist.

Die beiden Führungsschlitze können hierbei in einer gemeinsamen Führungswand des Basiselementes ausgebildet sein. Insbesondere ist es vorteilhaft, dass die Basiswand das Zentralelement des Kanülenoberteils und bevorzugt auch das Halteelement umschließend ausgebildet ist. Insbesondere ist eine Führungswand in Form eines Hohlzylinders für einen stabilen Aufbau vorteilhaft. Ebenso liegt es im Rahmen der Erfindung, dass das Basiselement mehrere Führungswände für das Kanülenoberteil und bevorzugt für das Halteelement aufweist.

Wie zuvor beschrieben, weist das Basiselement und/oder das Schiebelement zumindest eine Schräge auf, insbesondere eine Kulisse, die derart angeordnet ist, dass bei dem Injektionsvorgang in einem proximalen Endbereich bei Verschieben des Schiebeelementes in Injektionsrichtung eine Drehung des Schiebeelementes relativ zu dem Basiselement erfolgt. Hierdurch wird in konstruktiv einfacher Weise bei vollständig oder nahezu vollständig eingeführter Kanüle eine Drehung des Schiebeelementes erzielt, um das Arretierungselement zu lösen und den Ejektionsvorgang zu starten.

Vorteilhafterweise weisen daher Schiebeelement und Arretierungselement korrespondierende Anlageflächen auf, die derart angeordnet sind, dass durch Drehung des Schiebeelementes das Arretierungselement aus der Festlegestellung in die Ejektionsstellung überführbar ist. Bevorzugt weist das Arretierungselement einen Fortsatz und das Schiebeelement eine korrespondierende Führungsfläche auf.

Vorteilhafterweise weisen Basiselement und Schiebeelement korrespondierende Führungselemente auf, die derart ausgebildet und angeordnet sind, dass nur in dem proximalen Endbereich das Schiebeelement relativ zu dem Basiselement drehbar ist. Hierdurch wird vermieden, dass der Benutzer vor dem proximalen Endbereich bereits eine Drehung des Schiebeelementes relativ zu dem Basiselement durchführt, die zu einer Fehlfunktion und insbesondere zu einem vorzeitigen oder ausbleibenden Auslösen des Ejektionselementes führen könnte.

Vorteilhafterweise ist hierzu an einem der beiden Elemente, Basiselement und Schiebelement, ein Führungsschlitz oder eine Führungsnut ausgebildet, welche geradlinig in Injektionsrichtung verläuft, jedoch in dem proximalen Endbereich die Drehung des Schiebeelementes relativ zu dem Basiselement nachbildet. An dem anderen der beiden Elemente ist bevorzugt ein Führungsfortsatz ausgebildet, insbesondere ein Zapfen, welcher in die vorgenannte Führungsnut bzw. den Führungsschlitz eingreift und/oder diesen durchdringt.

Das Schiebeelement weist bevorzugt einen oder mehrere Führungsschlitze für den Fortsatz des Kanülenoberteils auf, die derart angeordnet sind, dass nach Drehen des Schiebeelementes in den proximalen Endbereich das Kanülenoberteil entgegen der Injektionsrichtung verschiebbar ist. Hierdurch ist in konstruktiv einfacher Weise ein Ejektionsvorgang möglich, ohne dass das Schiebeelement entgegen der Injektionsrichtung bewegt werden muss.

In einer vorteilhaften Ausgestaltung erfolgt somit bei dem Ejektionsvorgang ein Verschieben des Kanülenoberteils mit Kanüle entgegen der Injektionsrichtung relativ zu dem Basiselement und relativ zu dem Schiebeelement, sodass während des Ejektionsvorganges keine oder allenfalls eine geringfügige weitere Verschiebung zwischen Basiselement und Schiebeelement erfolgt.

Die zuvor genannte Schräge oder schräge Fläche ist bevorzugt an einem proximalen Ende der vorgenannten Führungsschlitze an dem Schiebeelement ausgebildet. Hierdurch ergibt sich ein konstruktiv einfacher Aufbau.

Vorteilhafterweise weist der Injektor eine Gegenkraftfeder auf, welche zwischen Basiselement und Schiebeelement einem Verschieben des Schiebeelementes in Injektionsrichtung entgegenwirkend angeordnet ist. Hierdurch ist sichergestellt, dass der Injektionsvorgang nur mittels Druck auf das Schiebelement gestartet wird und beispielsweise nicht bereits alleine aufgrund der Gewichtskraft des Schiebeelementes erfolgt. Hierbei zeigt sich ein weiterer Vorteil der vorliegenden Erfindung, diese Gegenkraftfeder der vorliegend beschriebenen vorteilhaften Ausführungsform kann gezielt auf das Erzeugen einer notwendigen Gegenkraft, die ein gleichmäßiges, kontinuierliches Verschieben des Schiebeelementes durch den Benutzer fördert, ausgelegt werden. Insbesondere ist es nicht notwendig, dass durch Komprimieren der Gegenkraftfeder bei Injektion gleichzeitig eine ausreichende Gegenkraft für die Ejektion durch die Gegenkraftfeder ausgebildet wird. Typischerweise wird die Gegenkraftfeder eine geringere Federkonstante aufweisen, gegenüber dem Ejektionselement und insbesondere einem als Ejektionsfeder ausgebildeten Ejektionselement.

Der erfindungsgemäße Injektor ist bevorzugt gemäß DE102018101275.6 ausgebildet.

Weitere bevorzugte Merkmale und Ausführungsformen werden im Folgenden anhand eines Ausführungsbeispiels und den Figuren erläutert. Dabei zeigt:
- Figur 1: eine Seitenansicht eines Ausführungsbeispiels eines erfindungsgemäßen Injektors zum transkutanen Einführen eines Sensors in einen Patienten;
- Figur 2: Seitenansichten von Kanüle, Kanülenoberteil und Halteelement des Injektors;
- Figur 3: eine Draufsicht von oben der Elemente gemäß Figur 2;
- Figur 4: Schnittdarstellungen der Elemente aus Figur 2;
- Figur 5: eine Schnittdarstellung des Injektors vor dem Injektionsvorgang;
- Figur 6: eine Schnittdarstellung des Injektors nach Beenden des Injektionsvorgangs;
- Figur 7: eine weitere Schnittdarstellung am Ende des Injektionsvorgangs, wobei die Schnittebene senkrecht zu der Schnittebene gemäß Figur 6 steht;
- Figur 8: eine Detailansicht des Detektors mit einem Ejektionsfederhalteelement;
- Figur 9: eine Detailansicht des Injektors im proximalen Endbereich des Injektionsvorgangs;
- Figur 10: eine Seitenansicht des Injektors nach Beenden des Ejektionsvorgangs,
- Figur 11: eine Schnittdarstellung der Ansicht gemäß Figur 9 und
- Figur 12: eine Schnittdarstellung der Ansicht gemäß Figur 10.

Gleiche Bezugszeichen in den Figuren bezeichnen gleiche bzw. gleichwirkende Elemente.

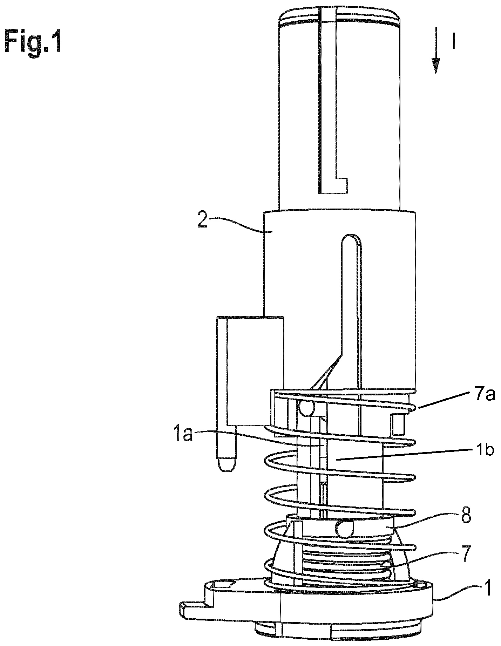

Figur 1 zeigt das Ausführungsbeispiel des erfindungsgemäßen Injektors in Seitenansicht. Der Injektor weist ein Basiselement 1 und ein Schiebeelement 2 auf. Das Schiebeelement ist in einer Injektionsrichtung I verschiebbar an dem Basiselement angeordnet.

Der Injektor kann zusätzlich ein Gehäuse aufweisen, welches an dem Basiselement angeordnet ist und das Basiselement sowie den unteren Teil, insbesondere die untere Hälfte des Schiebeelementes 2 gemäß Figur 1 umgibt. Aus Gründen der besseren Darstellbarkeit ist das Gehäuse in den Figuren nicht gezeigt.

Zur Verwendung des Injektors wird eine Basisplatte an die Haut des Patienten angeklebt und der Injektor mittels eines an der unteren Seite des Basiselementes ausgebildeten Bajonettverschlusses an die Basisplatte angebracht, so dass der Injektor lösbar an der Basisplatte und somit lösbar an dem Patienten angeordnet ist. Ebenso kann im Auslieferungszustand der Injektor bereits an der Basisplatte angebracht sein, so dass Injektor und Basisplatte an die Haut des Patienten angeklebt werden.

Das Gewebe des Patienten befindet sich somit bei allen Figuren an der unteren Seite, so dass in den Figuren die unteren Bereiche proximale Bereiche und die oberen Bereiche distale Bereiche zeigen.

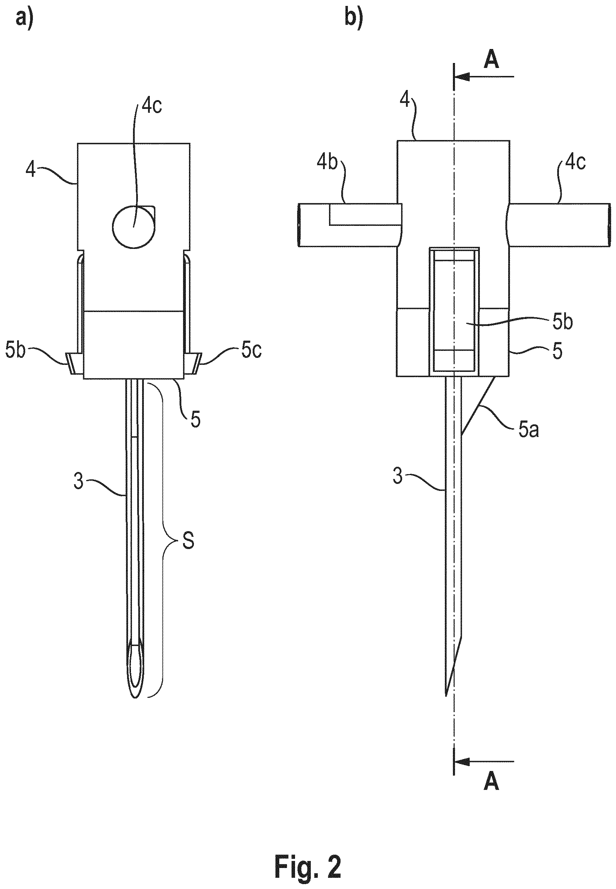

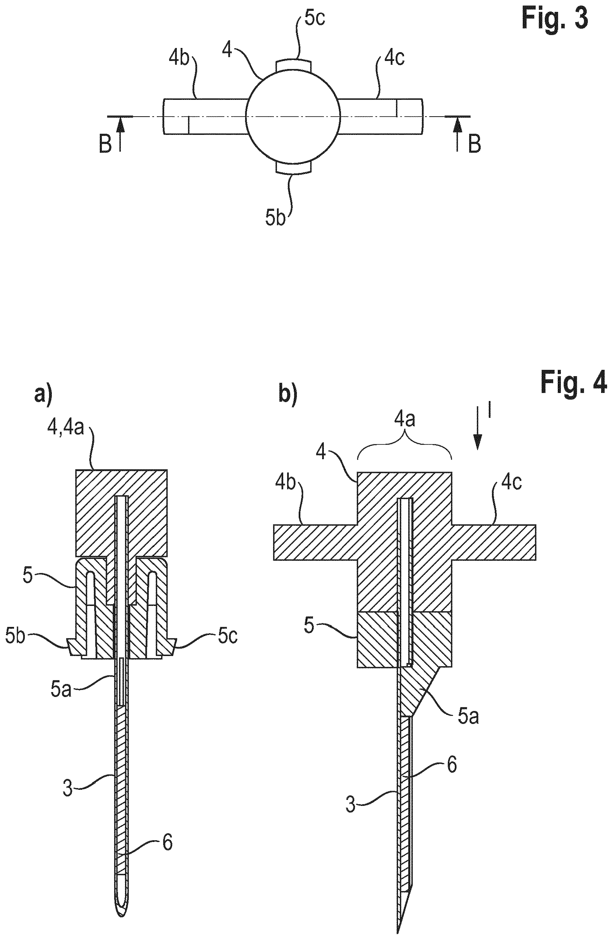

Das Basiselement weist einen in etwa als Hohlzylinder ausgebildeten Bereich auf, welcher eine Kanüle 3 mit einem Kanülenoberteil 4 und ein Halteelement 5 annähernd umschließt. Diese Elemente sind in den Figuren 2 bis 4 separat dargestellt:
Die Kanüle 3 ist an ihrem distalen Ende in einen Zentralelement 4a (siehe Figur 4b) des Kanülenoberteils 4 eingebettet und fest mit diesem verbunden. Unterhalb des Kanülenoberteils 4 ist das Halteelement 5 angeordnet, welches im distalen Bereich eine Einbuchtung aufweist, in welche das Kanülenoberteil 4 eingreift, wobei das Halteelement 5 mit leichtem Presssitz an dem Kanülenoberteil 4 angeordnet ist.

Das Kanülenoberteil 4 weist weiterhin zwei an gegenüberliegenden Seiten angeordnete Fortsätze 4b, 4c auf, welche sich senkrecht zur Längserstreckung der Kanüle 3 und somit senkrecht zur Injektionsrichtung I erstrecken.

Figur 2a zeigt hierbei eine Seitenansicht mit Draufsicht auf die Stirnseite des Fortsatzes 4c und Figur 2b zeigt eine Seitenansicht mit in der Zeichenebene liegenden Längserstreckung der Fortsätze 4b und 4c.

Figur 3 zeigt eine Draufsicht von oben auf das Kanülenoberteil 4. Figur 4a zeigt einen Schnitt gemäß der Schnittlinie A-A in Figur 2b, wobei die Schnittebene senkrecht zur Zeichenebene der Figur 2a steht. Figur 4b zeigt einen Schnitt gemäß der Schnittlinie B-B in Figur 3, wobei ebenfalls die Schnittebene senkrecht zur Zeichenebene gemäß Figur 3 steht.

Wie beispielsweise in Figur 2a ersichtlich, weist die Kanüle 3 in einem proximalem Bereich S einen Schlitz auf. Figur 2a zeigt die Draufsicht von vorne auf den Schlitz der Kanüle 3. In der Kanüle 3 ist ein Sensor 6 angeordnet, wie beispielsweise in den Figuren 4a und 4b ersichtlich.

Dieser Sensor soll transkutan in das Gewebe des Patienten mittels des Injektors eingebracht werden, um mittels eines als Detektor ausgebildeten Detektionselementes/Detektionseinheit optisch Messwerte zu ermitteln. Die Grundprinzipien einer solchen optische Messung sind in WO2016128334A1 und WO2006092317A1 beschrieben.

Ebenso ist das Einbringen anderer Sensoren, insbesondere von Sensoren mit Elektroden zur elektrischen Erfassung von Messwerten in gleicher Weise möglich.

Wie insbesondere in den Figuren 2b, 4a und 4b ersichtlich, weist das Halteelement 5 eine Nocke 5a auf, welche im Bereich des distalen Endes des Sensors 6 durch den Schlitz der Kanüle 3 in diese eingreift und somit am oberen, distalen Ende des Sensors 6 anliegt (siehe Figur 4b).

Das Halteelement 5 weist weiterhin als Rastnasen ausgebildete Festlegemittel 5b und 5c auf, so dass am Ende eines Injektionsvorgangs das Halteelement 5 selbsttätig an dem Basiselement arretierbar ist:

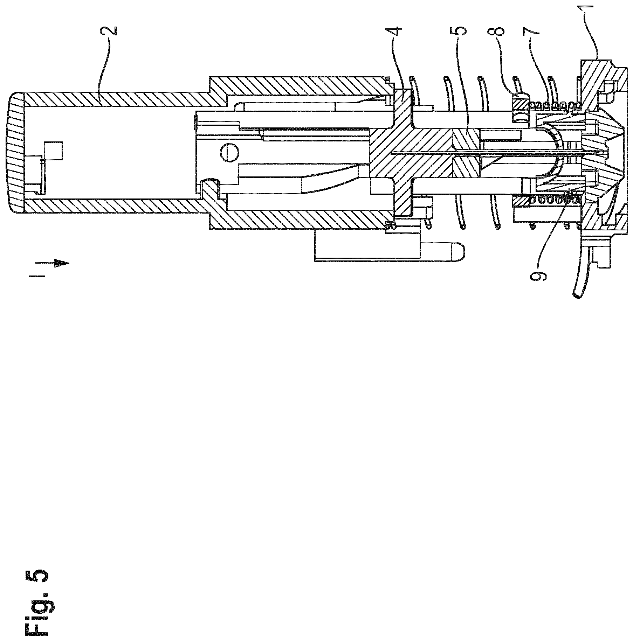

Figur 5 zeigt eine Schnittdarstellung des Injektors, wobei die Schnittebene durch die Zentralachse der Kanüle 3 und somit durch die Injektionsachse verläuft. Dargestellt ist der Zustand vor dem Injektionsvorgang, welcher den Auslieferungszustand darstellt: Kanüle und Sensor befinden sich innerhalb des Injektors, insbesondere innerhalb des Basiselementes 1.

Kanüle 3 mit Sensor 6, Halteelement 5 und Zentralelement 4a des Kanülenoberteils 4 sind innerhalb eines in etwa zylindrisch ausgebildeten Bereichs des Basiselementes 1 angeordnet. Dieser zylindrische Bereich weist geradlinig in Injektionsrichtung I verlaufende Führungsschlitze auf, von denen ein Führungsschlitz 1a in Figur 1 ersichtlich ist.

Die Fortsätze 4b und 4c des Kanülenoberteils 4 durchdringen den in etwa zylindrischen Bereich des Basiselements 1. Wie in Figur 5 ersichtlich, greift das Schiebeelement 2 außerhalb des zylindrischen Bereichs des Basiselementes 1 an den Fortsätzen 4b und 4c an. Das Schiebeelement 2 wird somit an einer Außenwand des zylindrischen Bereichs des Basiselements 1 in Injektionsrichtung verschiebbar geführt und Zentralelement 4a des Kanülenoberteils 4 sowie Halteelement 5 werden an der Innenseite des zylindrischen Bereichs des Basiselementes 1 in Injektionsrichtung verschiebbar geführt. Der zylindrische Bereich des Basiselements 1 bildet somit eine Führungswand 1b für diese Elemente.

Drückt der Benutzer nun das Schiebeelement 2 in Injektionsrichtung nach unten, so wird die Kraft über die Fortsätze 4b und 4c auf das Zentralelement 4a und somit auf die Kanüle 3 übertragen. Ebenso wird die Kraft über das Zentralelement 4a auf das Halteelement 5 übertragen, so dass diese Elemente sowie der Sensor 6 in Injektionsrichtung bewegt werden.

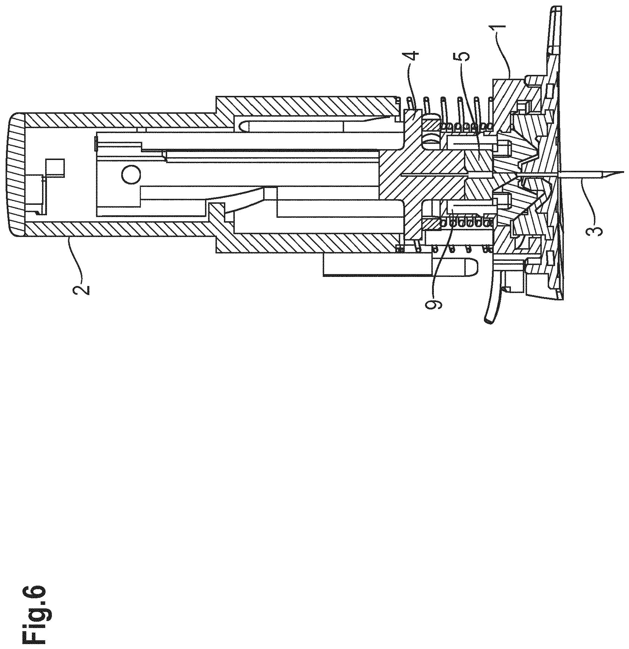

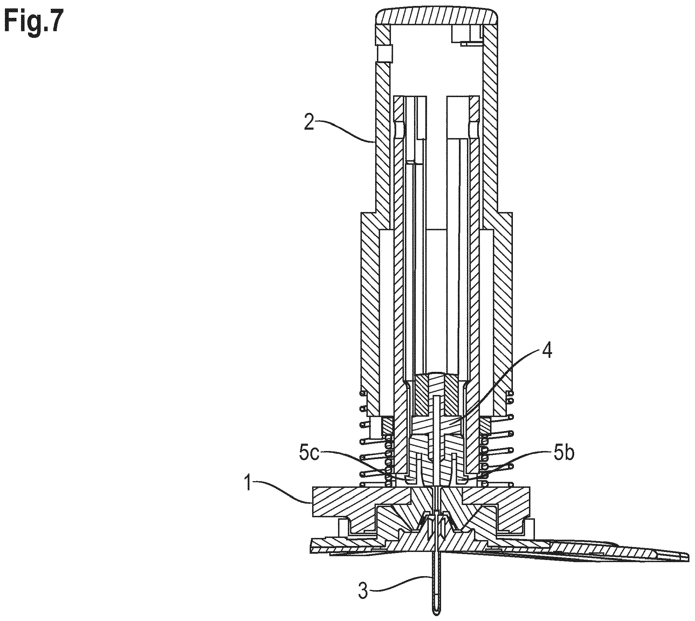

Figur 6 zeigt das Ende des Injektionsvorgangs: Das Schiebeelement 2 ist vollständig heruntergedrückt, die Kanüle 3 mit dem Sensor 6 ist transkutan in das Gewebe des Patienten eingedrungen und die als Rastnasen ausgebildeten Festlegemittel 5b und 5c sind in entsprechende Ausnehmungen des Basiselements 1 eingerastet, wie in Figur 7 ersichtlich.

Am Ende des Injektionsvorgangs ist somit das Halteelement 5 selbsttätig an dem Basiselement 1 festgelegt.

Bei dem nachfolgenden Ejektionsvorgang wird das Kanülenoberteil 4 entgegen der Injektionsrichtung I nach oben bewegt, so dass die Kanüle 3 aus dem Gewebe des Patienten herausgezogen wird. Da das Halteelement 5 jedoch an dem Basiselement 1 festgelegt ist, vollzieht das Halteelement diese Bewegung entgegen der Injektionsrichtung nicht mit. Ein eventuell vorhandener Presssitz zwischen Halteelement 5 und Kanülenoberteil 4 wird durch die Festlegung mittels der Rastnasen überwunden. Da weiterhin die Nocke 5a des Halteelements 5 in den Schlitz der Kanüle 3 an dem distalen Ende des Sensors in die Kanüle eingreift, wird hierdurch verhindert, dass bei einem Herausziehen der Kanüle 3 auch der Sensor aus dem Gewebe des Patienten herausgezogen wird. Insbesondere kann auch ein Anhaften oder eine Reibung zwischen Sensor und Kanüle hierdurch überwunden werden.

Nach Abschluss des Ejektionsvorgangs wird der Injektor von der zuvor genannten Basisplatte entfernt, so dass lediglich Basisplatte und Sensor 6 an dem Patienten verbleiben. Halteelement 5 und Sensor 5 sind somit als separate Einheiten ausgebildet.

Der Injektor gemäß dem vorliegenden Ausführungsbeispiel weist weiterhin ein als Ejektionsfeder 7 ausgebildetes Ejektionselement und ein als Ejektionsfederhalteelement 8 ausgebildetes Arretierungselement für das Ejektionselement auf. Wie in Figur 1 ersichtlich, ist im Auslieferungszustand die Ejektionsfeder 7 komprimiert zwischen Basiselement 1 und Ejektionsfederhalteelement 8 angeordnet. Die Ejektionsfeder 7 umschließt den zylindrischen Bereich des Basiselementes 1 in einem proximalen Bereich.

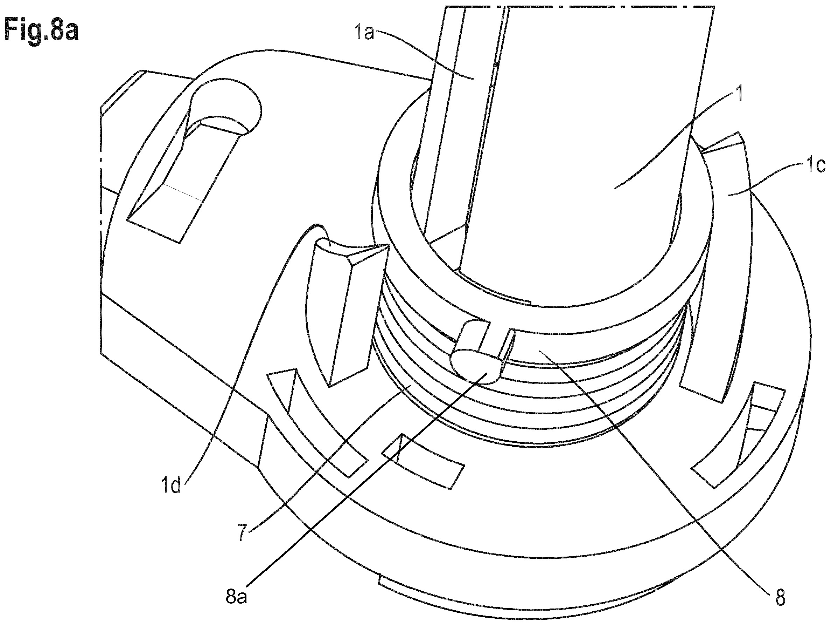

Das Ejektionsfederhalteelement 8 ist im Wesentlichen ringförmig ausgebildet und weist sowohl an der Innenseite, als auch an der Außenseite einen Zapfen auf. Mittels des inneren Zapfens ist das Ejektionsfederhalteelement an einer senkrecht zur Injektionsrichtung verlaufenden Führung des Basiselementes lösbar festgelegt, siehe Figur 8a. In dem durch einen Kreis in Figur 8a markierten Bereich greift der innere Zapfen des Ejektionsfederhalteelements 8 in eine Führung an der Außenwand des Basiselementes 1 ein, so dass in diesem Zustand keine Expansion der Ejektionsfeder 7 möglich ist. Wie nachfolgend näher erläutert, sind Schiebeelement 2 und Ejektionsfederhalteelement 8 derart zusammenwirkend ausgebildet, dass am Ende des Injektionsvorgangs eine Drehung des Ejektionsfederhalteelementes 8 erfolgt, so dass der Zapfen des Ejektionsfederhalteelementes 8 in der Darstellung gemäß Figur 8a nach links gedreht wird und so in den Bereich des Führungsschlitzes 1a des Basiselementes 1 gelangt, so dass eine Expansion der Ejektionsfeder 7 möglich ist.

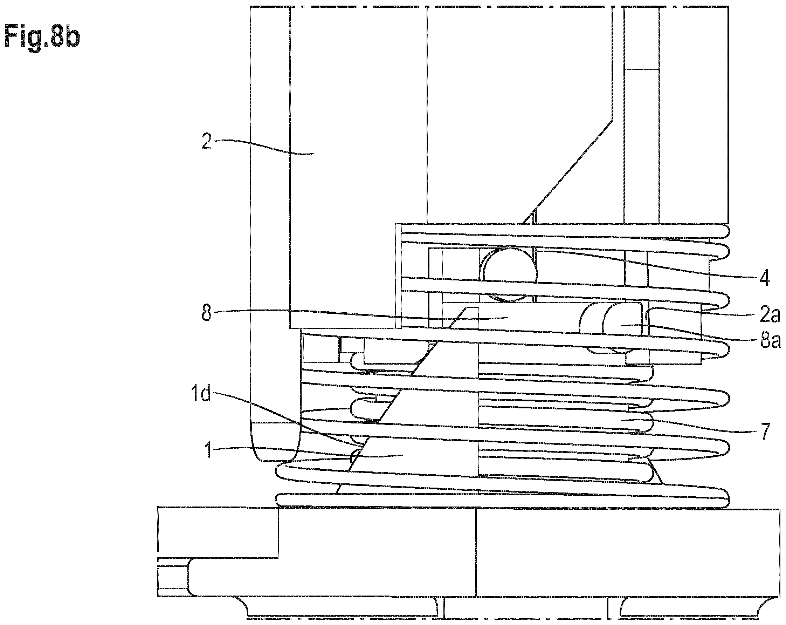

Wie in den Figuren 8a und 8b ersichtlich, weist das Basiselement in einem proximalen Bereich zwei als Schräge, d. h. schräge Flächen, ausgebildete Führungen 1c und 1d welche am Ende des Injektionsvorgangs mit korrespondierenden Anlageflächen des Schiebeelementes 2 in Kontakt gelangen. Wird in diesem Endbereich das Schiebeelement weiter heruntergedrückt, so erfolgt aufgrund der Schrägen 1c und 1d eine Drehung des Schiebeelementes relativ zu dem Basiselement um die Injektionsachse. Zur besseren Verdeutlichung sind in Figur 8a Elemente wie beispielsweise das Schiebeelement nicht dargestellt.

Wie in Figur 8b ersichtlich, weist das Ejektionsfederhalteelement 8 einen als Zapfen ausgebildeten äußeren Fortsatz 8a auf, welcher bei Drehen des Schiebeelementes 2 mit einer korrespondierenden Führungsfläche 2a des Schiebeelementes 2 in Kontakt kommt, so dass eine Drehung des Ejektionsfederhalteelementes 8 wie zuvor beschrieben erfolgt.

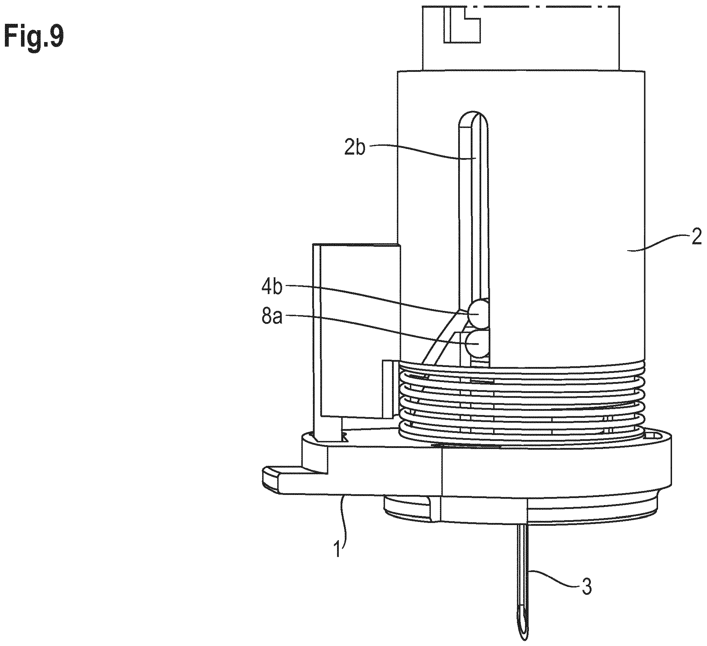

Figur 9 zeigt den Zustand mit vollständig nach unten gedrücktem Schiebeelement 2, so dass auch die Drehung des Schiebeelementes 2 relativ zu dem Basiselement 1 beendet ist. Wie ersichtlich, befinden sich in diesem Endzustand des Injektionsvorgangs sowohl der äußere Zapfen 8a des Ejektionsfederhalteelementes 8, als auch der Fortsatz 4b des Kanülenoberteils 4 im Bereich eines Führungsschlitzes 2b des Schiebeelementes 2. Auf der gegenüberliegenden Seite (nicht ersichtlich) befindet sich entsprechend der Fortsatz 4c des Kanülenoberteils 4 in einem radial gegenüberliegend angeordneten Führungsschlitz des Schiebeelementes 2.

In diesem Zustand besteht somit für die Fortsätze 4b und 4c sowie für den äu-βeren Zapfen 8a keine Begrenzung hinsichtlich einer Bewegung entgegen der Injektionsrichtung. Im Ergebnis erfolgt eine Expansion der Ejektionsfeder 7, wodurch Ejektionsfederhalteelement 8 und Kanülenoberteil 4 in einem Ejektionsvorgang entgegen der Injektionsrichtung nach oben gedrückt werden. Das Halteelement 5 hingegen ändert aufgrund der eingerasteten Halteelemente die Position nicht.

Es erfolgt somit ein Herausziehen der Kanüle 3 aus dem Gewebe des Patienten, wobei der Sensor 6 durch die Nocke 5a des Haltelementes 5 an einem Herausziehen gehindert wird.

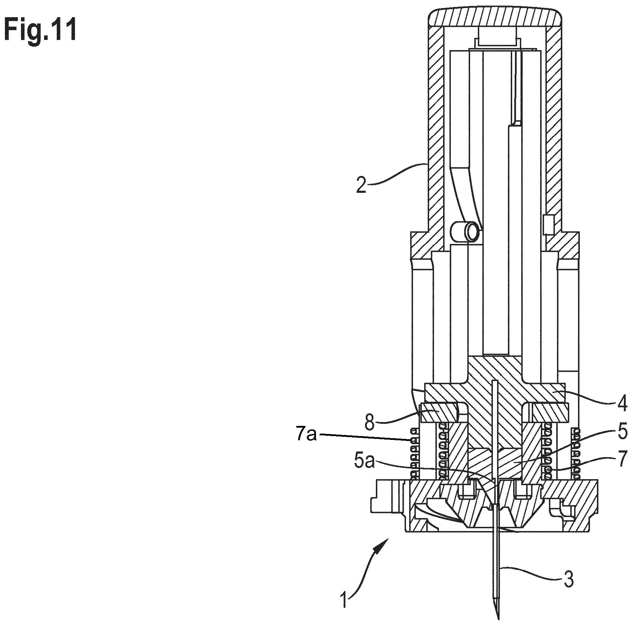

In Figur 11 ist die Konstellation aus Figur 9 als Schnittbild dargestellt, wobei die Injektionsachse innerhalb der Schnittebene liegt und der Schnitt entlang der Schnittlinie B gemäß Figur 3 verläuft. Hier ist insbesondere die komprimierte Ejektionsfeder 7 ersichtlich, welche an dem Halteelement 8 einerseits und dem Basiselement 1 andererseits angreift. Konzentrisch zu der Ejektionsfeder 7, jedoch mit größerem Radius ist eine Gegenkraftfeder 7a angeordnet, welche an dem Basiselement 1 einerseits und Schiebeelement 2 andererseits angreift. Auch diese Gegenkraftfeder ist in dieser Konstellation vollständig komprimiert. Die Gegenkraftfeder dient insbesondere dem Zweck, ein Herabsinken des Schiebeelementes 2 aufgrund der Schwerkraft zu vermeiden sowie dem Benutzer bei dem Injektionsvorgang eine in etwa konstante Gegenkraft zu bieten, um eine gleichmäßige Injektion zu ermöglichen, insbesondere eine gleichmäßige Geschwindigkeit des Eindringens der Kanüle 3 in den Patienten.

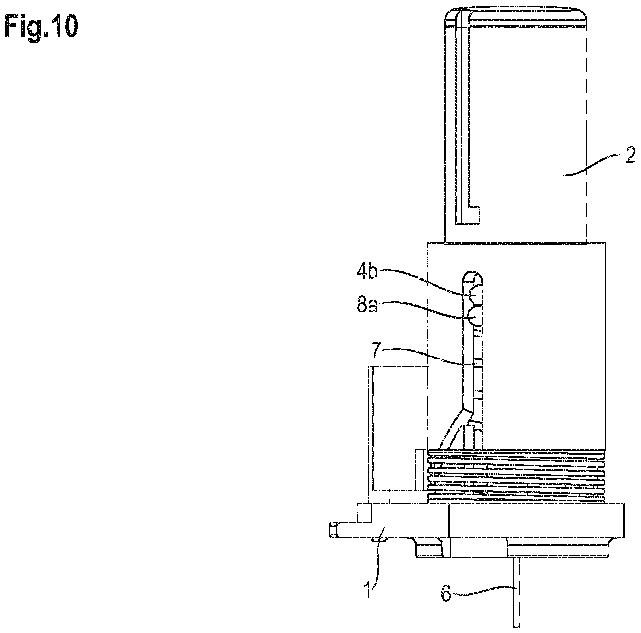

In Figur 10 ist die Ansicht gemäß Figur 9 dargestellt, jedoch nach Beenden des Ejektionsvorgangs:
Wie zuvor beschrieben, wird am Ende des Injektionsvorgangs das Halteelement 8 um die Injektionsachse gedreht, so dass es aus einer Festlegestellung, bei welcher keine Bewegung des Halteelementes in Ejektionsrichtung, d. h. entgegen der Injektionsrichtung I möglich ist in eine Ejektionsstellung überführt wird, in welcher eine Bewegung in Ejektionsrichtung erfolgen kann.

Wie bereits zu Figur 8a beschrieben, weist das Halteelement 8 an der Innenseite einen Zapfen auf, welcher gemäß der in Figur 8a dargestellten Festlegestellung in einem waagerecht verlaufenden Schlitz des Basiselementes 1 angeordnet ist, so dass keine Bewegung in Ejektionsrichtung (in Figur 8a nach oben) möglich ist.

Am Ende des Injektionsvorgangs erfolgt eine Drehung des Halteelementes 8 um die Injektionsachse, gemäß Figur 8a im Uhrzeigersinn, so dass der innere Zapfen des Halteelementes 8 in der Flucht des Führungsschlitzes 1a des Basiselementes 1 zu liegen kommt. Hierdurch ist somit eine Bewegung des Führungselementes 8 in Ejektionsrichtung (nach oben) möglich. Diese Drehung des Halteelementes 8 erfolgt, da am Ende des Injektionsvorgangs aufgrund der Schrägen 1d und 1c des Basiselementes 1 und der korrespondierenden Schrägen des Schiebeelementes 2 eine Drehung des Schiebeelementes um die Injektionsachse (vorliegend im Uhrzeigersinn) erfolgt und durch die Fläche 2a des Schiebeelementes 2, welche an dem äußeren Zapfen des Halteelementes 8 angreift, die Drehung auf das Halteelement 8 übertragen wird.

Befindet sich das Halteelement 8 in Ejektionsstellung, so ist eine Expansion der Expansionsfeder 7 möglich: Hierdurch wird das Halteelement 8 nach oben gedrückt und diese Bewegung auf das Kanülenoberteil 4 und somit auch die Kanüle 3 übertragen, so dass die Kanüle 3 in einem Ejektionsvorgang aus dem Patienten herausgezogen wird. Aufgrund des zuvor beschriebenen Halteelementes 5 verbleibt der Sensor 6 jedoch transkutan in dem Patienten.

Figur 10 zeigt nun die Situation nach Beenden des Ejektionsvorgangs: Die Ejektionsfeder 7 befindet sich in einem expandierten Zustand, Halteelement 8 und Kanülenoberteil 4 (sowie Kanüle 3) sind nach oben geschoben und befinden sich innerhalb des Basiselementes 1. Die Position des Schiebeelementes 2 ist jedoch unverändert, so dass nach wie vor die außenliegende Gegenkraftfeder sich in einem komprimierten Zustand befindet.

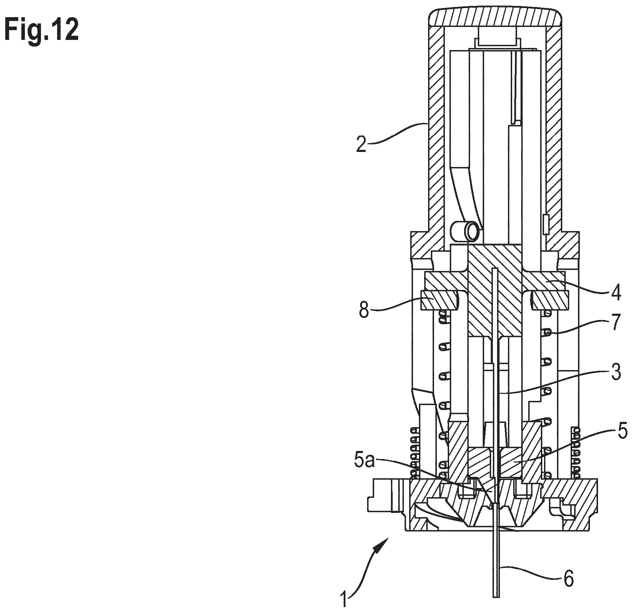

In Figur 12 ist ein Schnitt analog zu Figur 11 dargestellt, jedoch am Ende des Ejektionsvorgangs gemäß Figur 10.

Der Injektor gemäß dem vorliegenden Ausführungsbeispiel weist weiterhin eine Kanülenführung 9 für die Kanüle 3 auf, wie insbesondere in den Figuren 5 und 6 ersichtlich:
Die Kanülenführung 9 dient dazu, die Kanüle insbesondere während des Injektionsvorgangs, jedoch auch während des Ejektionsvorgangs zu führen, um ein Verkippen oder seitliches Verrutschen zu vermeiden. Hierzu ist die Kanülenführung 9 an zwei gegenüberliegenden Seiten an dem Basiselement 1 angeordnet und weist mittig eine elastische Führungsfläche mit einer Öffnung auf, welche durch die Kanüle 3 durchdrungen wird und die Kanülenführung 9 in zwei Hälften unterteilt. In Figur 5 ist der Zustand vor Beginn der Injektion dargestellt. Hier wird die Kanüle 3 in einem proximalen Bereich durch die Kanülenführung 9 geführt, so dass bei der nachfolgenden Injektion ein Verkippen oder seitliches Verschieben vermieden wird.

Nach Beenden des Injektionsvorgangs erreicht das Halteelement 5 und das Kanülenoberteil 4 den Bereich der Kanülenführung 9. Aufgrund der Zweiteilung der Kanülenführung 9 können die elastischen Elemente der Kanülenführung 9 durch das Halteelement 5 nach rechts und links gedrückt werden. Dies ist in Figur 6 ersichtlich.

## Patentansprüche

1. Injektor zum transkutanen Einführen eines Sensors (4) in einen Patienten, mit einer Kanüle (3), einem Basiselement (1), einem verschiebbar an dem Basiselement (1) angeordneten Schiebeelement (2) zum transkutanen Einführen der Kanüle (3) in den Patienten in einer Injektionsrichtung (I) und mit einem Ejektionselement (7) zum selbsttätigen Herausziehen der Kanüle (3) aus dem Patienten entgegen der Injektionsrichtung (I) mittels des Ejektionselementes (7) in einem Ejektionsvorgang, wobei der Injektor ein Arretierungselement (8) für das Ejektionselement (7) aufweist, so dass in einem Auslieferungszustand das Ejektionselement (7) in einem energiegeladenen Zustand arretierbar ist und
Schiebeelement (2) und Arretierungselement (8) mittelbar oder unmittelbar zusammenwirkend ausgebildet sind, um in einem Injektionszustand bei in den Patienten transkutan eingeführter Kanüle (3) die Arretierung des Ejektionselementes (7) zu lösen, um den Ejektionsvorgang automatisch zu starten,
wobei das Arretierungselement (8) in einer Festlegestellung an dem Basiselement (1) festlegbar ausgebildet ist und in einer Ejektionsstellung entgegen der Injektionsrichtung (I) an dem Basiselement (1) verschiebbar ausgebildet ist, **dadurch gekennzeichnet,**
**dass** das Arretierungselement (8) drehbar an dem Basiselement (1) angeordnet ist und mittels Drehung des Arretierungselements (8) aus der Festlegestellung in die Ejektionsstellung überführbar ausgebildet ist.

2. Injektor nach Anspruch 1,
**dadurch gekennzeichnet,**
**dass** die Kanüle (3) in einem distalen Endbereich an einem Kanülenoberteil (4) des Injektors angeordnet ist,
**dass** das Kanülenoberteil (4) in Injektionsrichtung (I) verschiebbar an dem Basiselement (1) angeordnet ist und
**dass** das Ejektionselement (7) mit dem Kanülenoberteil (4) zusammenwirkend ausgebildet ist, so dass mittels des Ejektionselementes (7) das Kanülenoberteil (4) entgegen der Injektionsrichtung (I) verschiebbar ist.

3. Injektor nach Anspruch 1 oder 2,
**dadurch gekennzeichnet,**
**dass** das Arretierungselement (8) um eine Injektionsachse drehbar an dem Basiselement (1) angeordnet ist.

4. Injektor nach zumindest den Ansprüchen 1 und 2,
**dadurch gekennzeichnet,**
**dass** das Arretierungselement (8) zwischen Ejektionselement (7) und Kanülenoberteil (4) angeordnet ist, so dass mittels des Ejektionselements (7) eine Kraft in Ejektionsrichtung über das Arretierungselement (8) auf das Kanülenoberteil (4) übertragbar ist.

5. Injektor nach einem der vorangegangenen Ansprüche,
**dadurch gekennzeichnet,**
**dass** das Ejektionselement (7) als Ejektionsfeder (7) ausgebildet ist.

6. Injektor nach Anspruch 5,
**dadurch gekennzeichnet,**
**dass** das die Ejektionsfeder (7) mittels des Arretierungselementes (8) in einem gespannten oder bevorzugt komprimierten Zustand festlegbar ist.

7. Injektor nach Anspruch 2,
**dadurch gekennzeichnet,**
**dass** das Kanülenoberteil (4) ein Zentralelement (4a), an welchem die Kanüle (3) angeordnet und mindestens einen Führungsfortsatz (4b, 4c) aufweist und das Basiselement (1) mindestens eine Führungswand (1b) mit einem Führungsschlitz (1a) für den Führungsfortsatz (4b, 4c) des Kanülenoberteils (4) aufweist, zur Führung des Kanülenoberteils (4) in Injektionsrichtung (I), insbesondere,
**dass** der Führungsfortsatz (4b, 4c) die Führungswand (1b) des Basiselements durchdringt und das Schiebeelement (2) an der dem Zentralelement (4a) des Kanülenoberteils (4) abgewandten Seite der Führungswand (1b) an dem Führungsfortsatz (4b, 4c) angreifend ausgebildet ist.

8. Injektor nach Anspruch einem der vorangegangenen Ansprüche,
**dadurch gekennzeichnet,**
**dass** Basiselement (1) und/oder Schiebelement (2) zumindest eine Schräge (1c, 1d) aufweisen, die derart angeordnet ist, dass beim Injektionsvorgang in einem proximalen Endbereich bei Verschieben des Schiebelementes (2) in Injektionsrichtung (I) eine Drehung des Schiebelements (2) relativ zu dem Basiselement (1) erfolgt.

9. Injektor nach Anspruch 8,
**dadurch gekennzeichnet,**
**dass** Schiebeelement (2) und Arretierungselement (8) korrespondierende Anlageflächen aufweisen, die derart angeordnet sind, dass durch Drehung des Schiebelements (2) das Ejektionselement (7) aus einer Festlegestellung lösbar ist, insbesondere, dass das Arretierungselement (8) einen Fortsatz (8a, 8b) und das Schiebeelement (2) eine korrespondierende Führungsfläche aufweist.

10. Injektor nach einem der Ansprüche 8 bis 9,
**dadurch gekennzeichnet,**
**dass** Basiselement (1) und Schiebelement (2) korrespondierende Führungselemente (1a, 2b) aufweisen, die derart ausgebildet und angeordnet sind, dass nur in dem proximalen Endbereich das Schiebeelement (2) relativ zu dem Basiselement (1) drehbar ist.

11. Injektor nach Anspruch 2 und 10,
**dadurch gekennzeichnet,**
**dass** das Schiebelement (2) Führungsschlitze (2b) für den Führungsfortsatz (4b, 4c) des Kanülenoberteils (4) aufweist, die derart angeordnet sind, dass nach Drehen des Schiebelementes (2) in dem proximalen Endbereich das Kanülenoberteil (4) entgegen der Injektionsrichtung (I) verschiebbar ist.

12. Injektor nach Anspruch 11,
**dadurch gekennzeichnet,**
**dass** die Schräge (1c, 1d) an einem proximalen Ende der Führungsschlitze (2b) an dem Schiebelement (2) ausgebildet ist.

13. Injektor nach einem der vorangegangenen Ansprüche,
**dadurch gekennzeichnet,**
**dass** der Injektor eine Gegenkraftfeder aufweist, welche zwischen Basiselement (1) und Schiebelement (2) einem Verschieben des Schiebelementes (2) in Injektionsrichtung (I) entgegenwirkend angeordnet ist.

## Claims

1. Injector for transcutaneous introduction of a sensor (4) into a patient, having a cannula (3), a base element (1), a sliding element (2) displaceably arranged on the base element (1) for transcutaneous introduction of the cannula (3) into the patient in an injection direction (I) and having an ejection element (7) for automatic withdrawal of the cannula (3) from the patient in a direction opposite to the injection direction (I) by means of the ejection element (7) in an ejection operation, wherein the injector has a locking element (8) for the ejection element (7), so that in a delivery state the ejection element (7) is lockable in an energy-loaded state and
the sliding element (2) and the locking element (8) are designed to indirectly or directly co-operate in order that, in an injection state in which the cannula (3) has been transcutaneously introduced into the patient, the locking of the ejection element (7) is released in order automatically to start the ejection operation, wherein the locking element (8) is designed to be fixable on the base element (1) in a fixing position and designed so as to be displaceable on the base element (1) in a direction opposite to the injection direction (I) in an ejection position,
**characterised in that**
the locking element (8) is rotatably arranged on the base element (1) and is designed so as to be displaceable from the fixing position to the ejection position by rotation of the locking element (8).

2. Injector according to claim 1,
**characterised in that**
the cannula (3) is arranged in a distal end region on a cannula upper part (4) of the injector;
the cannula upper part (4) is arranged on the base element (1) so to be displaceable in the injection direction (I); and
the ejection element (7) is designed to co-operate with the cannula upper part (4) so that, by means of the ejection element (7), the cannula upper part (4) is displaceable in a direction opposite to the injection direction (I).

3. Injector according to claim 1 or 2,
**characterised in that**
the locking element (8) is arranged on the base element (1) so as to be rotatable about an injection axis.

4. Injector according to at least claims 1 and 2,
**characterised in that**
the locking element (8) is arranged between the ejection element (7) and the cannula upper part (4) so that, by means of the ejection element (7), a force is transmissible to the cannula upper part (4) in the ejection direction via the locking element (8).

5. Injector according to any one of the preceding claims,
**characterised in that**
the ejection element (7) is configured as an ejection spring (7).

6. Injector according to claim 5,
**characterised in that**
the ejection spring (7) is fixable in a tensioned or, preferably, compressed state by means of the locking element (8).

7. Injector according to claim 2,
**characterised in that**
the cannula upper part (4) has a central element (4a) on which the cannula (3) is arranged and has at least one guide projection (4b, 4c), and the base element (1) has at least one guide wall (1b) with a guide slot (1a) for the guide projection (4b, 4c) of the cannula upper part (4), for guiding the cannula upper part (4) in the injection direction (I);
especially, the guide projection (4b, 4c) passes through the guide wall (1b) of the base element, and the sliding element (2) is designed to act on the guide projection (4b, 4c) on the side of the guide wall (1b) that faces away from the central element (4a) of the cannula upper part (4).

8. Injector according to any one of the preceding claims,
**characterised in that**
the base element (1) and/or the sliding element (2) have at least one sloping surface (1c, 1d) which is arranged in such a way that, during the injection operation, on displacement of the sliding element (2) in the injection direction (I) the sliding element (2) rotates relative to the base element (1) in a proximal end region.

9. Injector according to claim 8,
**characterised in that**
the sliding element (2) and the locking element (8) have complementary contact faces which are arranged in such a way that, by rotation of the sliding element (2), the ejection element (7) is releasable from a fixing position; especially, the locking element (8) has a projection (8a, 8b) and the sliding element (2) has a complementary guide face.

10. Injector according to either one of claims 8 and 9,
**characterised in that**
the base element (1) and the sliding element (2) have complementary guide elements (1a, 2b) which are configured and arranged in such a way that the sliding element (2) is rotatable relative to the base element (1) only in the proximal end region.

11. Injector according to claim 2 and 10,
**characterised in that**
the sliding element (2) has guide slots (2b) for the guide projection (4b, 4c) of the cannula upper part (4), which guide slots are arranged in such a way that, after rotation of the sliding element (2) in the proximal end region, the cannula upper part (4) is displaceable in a direction opposite to the injection direction (I).

12. Injector according to claim 11,
**characterised in that**
the sloping surface (1c, 1d) is formed on the sliding element (2) at a proximal end of the guide slots (2b).

13. Injector according to any one of the preceding claims,
**characterised in that**
the injector has a counter-force spring which is arranged between the base element (1) and the sliding element (2) so as to counteract displacement of the sliding element (2) in the injection direction (I).

## Revendications

1. Injecteur pour l'introduction transcutanée d'un capteur (4) dans un patient, avec une canule (3), un élément de base (1), un élément coulissant (2) disposé de manière coulissante sur l'élément de base (1) pour l'introduction transcutanée de la canule (3) dans le patient dans une direction d'injection (I) et avec un élément d'éjection (7) pour l'extraction automatique de la canule (3) du patient à l'encontre de la direction d'injection (I) au moyen de l'élément d'éjection (7) dans un processus d'éjection, dans lequel l'injecteur présente un élément de blocage (8) pour l'élément d'éjection (7), de sorte que dans un état de distribution l'élément d'éjection (7) peut être bloqué dans un état chargé en énergie et
l'élément coulissant (2) et l'élément de blocage (8) sont réalisés de manière à coopérer indirectement ou directement, afin, dans un état d'injection, de libérer le blocage de l'élément d'éjection (7) lorsque la canule (3) est introduite de manière transcutanée dans le patient, afin de démarrer automatiquement le processus d'éjection,
dans lequel l'élément de blocage (8) dans une position de fixation est réalisé de manière à pouvoir être fixé sur l'élément de base (1) et dans une position d'éjection est réalisé de manière à pouvoir coulisser sur l'élément de base (1) à l'encontre de la direction d'injection (I), **caractérisé en ce**
**que** l'élément de blocage (8) est disposé de manière rotative sur l'élément de base (1) et est réalisé de manière à pouvoir être amené de la position de fixation dans la position d'éjection au moyen de la rotation de l'élément de blocage (8).

2. Injecteur selon la revendication 1,
**caractérisé en ce**
**que** la canule (3) est disposée dans une zone d'extrémité distale sur une partie supérieure de canule (4) de l'injecteur,
**que** la partie supérieure de canule (4) est disposée sur l'élément de base (1) de manière à pouvoir coulisser dans la direction d'injection (I) et
**que** l'élément d'éjection (7) est réalisé de manière à coopérer avec la partie supérieure de canule (4), de sorte que la partie supérieure de canule (4) peut coulisser à l'encontre de la direction d'injection (I) au moyen de l'élément d'éjection (7).

3. Injecteur selon la revendication 1 ou 2,
**caractérisé en ce**
**que** l'élément de blocage (8) est disposé sur l'élément de base (1) de manière à pouvoir tourner autour d'un axe d'injection.

4. Injecteur selon au moins l'une des revendications 1 et 2,
**caractérisé en ce**
**que** l'élément de blocage (8) est disposé entre l'élément d'éjection (7) et la partie supérieure de canule (4), de sorte qu'au moyen de l'élément d'éjection (7) une force dans la direction d'éjection peut être transmise sur la partie supérieure de canule (4) par l'intermédiaire de l'élément de blocage (8).

5. Injecteur selon l'une des revendications précédentes,
**caractérisé en ce**
**que** l'élément d'éjection (7) est réalisé en tant que ressort d'éjection (7).

6. Injecteur selon la revendication 5,
**caractérisé en ce**
**que** le ressort d'éjection (7) peut être fixé dans un état tendu ou de préférence comprimé au moyen de l'élément de blocage (8).

7. Injecteur selon la revendication 2,
**caractérisé en ce**
**que** la partie supérieure de canule (4) présente un élément central (4a), sur lequel la canule (3) est disposée et au moins un prolongement de guidage (4b, 4c) et l'élément de base (1) présente au moins une paroi de guidage (1b) avec une fente de guidage (1a) pour le prolongement de guidage (4b, 4c) de la partie supérieure de canule (4), pour le guidage de la partie supérieure de canule (4) dans la direction d'injection (I),
en particulier
**que** le prolongement de guidage (4b, 4c) traverse la paroi de guidage (1b) de l'élément de base et l'élément coulissant (2) est réalisé de manière à agir sur la face de la paroi de guidage (1b) opposée à l'élément central (4a) de la partie supérieure de canule (4) sur le prolongement de guidage (4b, 4c).

8. Injecteur selon l'une des revendications précédentes,
**caractérisé en ce**
**que** l'élément de base (1) et/ou l'élément coulissant (2) présentent au moins un chanfrein (1c, 1d), qui est disposé de telle sorte que lors du processus d'injection une rotation de l'élément coulissant (2) par rapport à l'élément de base (1) s'effectue dans une zone d'extrémité proximale lors du coulissement de l'élément coulissant (2) dans la direction d'injection (I).

9. Injecteur selon la revendication 8,
**caractérisé en ce**
**que** l'élément coulissant (2) et l'élément de blocage (8) présentent des surfaces d'appui correspondantes, qui sont disposées de telle sorte que par la rotation de l'élément coulissant (2) l'élément d'éjection (7) peut être libéré d'une position de fixation, en particulier que l'élément de blocage (8) présente un prolongement (8a, 8b) et l'élément coulissant (2) une surface de guidage correspondante.

10. Injecteur selon l'une des revendications 8 à 9,
**caractérisé en ce**
**que** l'élément de base (1) et l'élément coulissant (2) présentent des éléments de guidage (1a, 2b) correspondants, qui sont réalisés et disposés de telle sorte que l'élément coulissant (2) peut être amené en rotation par rapport à l'élément de base (1) uniquement dans la zone d'extrémité proximale.

11. Injecteur selon la revendication 2 et 10,
**caractérisé en ce**
**que** l'élément coulissant (2) présente des fentes de guidage (2b) pour le prolongement de guidage (4b, 4c) de la partie supérieure de canule (4), qui sont disposées de telle sorte qu'après la rotation de l'élément coulissant (2) dans la zone d'extrémité proximale la partie supérieure de canule (4) peut coulisser à l'encontre de la direction d'injection (I).

12. Injecteur selon la revendication 11,
**caractérisé en ce**
**que** le chanfrein (1c, 1d) est réalisé sur une extrémité proximale des fentes de guidage (2b) sur l'élément coulissant (2).

13. Injecteur selon l'une des revendications précédentes,
**caractérisé en ce**
**que** l'injecteur présente un ressort à force antagoniste, lequel est disposé entre l'élément de base (1) et l'élément coulissant (2) de manière à pouvoir s'opposer à un coulissement de l'élément coulissant (2) dans la direction d'injection (I).
